# EUROPÄISCHE PATENTSCHRIFT

(11) **EP 0 825 264 B1**
(45) Veröffentlichungstag und Bekanntmachung des Hinweises auf die Patenterteilung: **13.10.2004**
(21) Anmeldenummer: 97113807.8
(22) Anmeldetag: 09.08.1997
(51) Int. Cl.: C12Q 1/42, G01N 33/52

(54) **Stabile Mischung zum Nachweis alkalischer Phosphatase mit einem Salz der o-Kresolphthaleinmonophosphorsäure**
Stable mixture for detecting alkaline phosphatase, with a salt of o-cresolphthalein monophosphoric acid
Mélange stable pour la détection de phosphatase alcaline, avec un sel du dérivé acide monophosphorique de o-cresolphtaléine

(30) Priorität: 12.08.1996 DE 19632432
(43) Veröffentlichungstag der Anmeldung: 25.02.1998
(73) Patentinhaber: Roche Diagnostics GmbH, 68298 Mannheim (DE)
(72) Erfinder: Nagel, Rolf, 68642 Bürstadt (DE); Bossert-Reuther, Steffen, 69126 Heidelberg (DE); Zeibig, Thomas, Dr., 67125 Dannstadt-Schauernheim (DE)

(56) Entgegenhaltungen:
- EP-A- 0 182 179
- US-A- 3 975 405
- CHEMICAL ABSTRACTS, vol. 125, no. 1, 1.Juli 1996 Columbus, Ohio, US; abstract no. 4404g, LION CORP.: "Test Paper Containing phenolphthalein monophosphates..." Seite 4410; Spalte 2; XP002046345

## Beschreibung

Die Erfindung betrifft eine stabile Mischung zum alkalischen Phosphatasenachweis, enthaltend ein Salz der o-Kresolphthaleinmonophosphorsäure. Gegenstand der Erfindung ist auch ein Analysenelement zum Nachweis alkalischer Phosphatase, enthaltend eine Matrix mit einem Substrat des Enzyms alkalischer Phosphatase, das in Anwesenheit des Enzyms zu einer Farb-Änderung führt. Außerdem ist Gegenstand der Erfindung ein Verfahren zur Bestimmung von alkalischer Phosphatase mit einem Salz der o-Kresolphthaleinmonophosphorsäure als Enzymsubstrat und einem Phosphorsäureakzeptor.

Die Bestimmung der alkalischen Phosphataseaktivität in menschlicher Körperflüssigkeit besitzt eine sehr große klinische Bedeutung. US-Patent 3,975,405 beschreibt die Verwendung von o-Kresolphthaleinmonophosphorsäure-Salzen als Substrate für die Bestimmung von alkalischer Phosphatase. Als bevorzugte Salze werden Alkali-, Erdalkali- und Ammoniumsalze sowie Salze von protonierten organischen Aminen genannt. Beispiele für solche protonierten organischen Amine sind Cyclohexylammonium, Tetramethylethylendiammonium und Ethanolammonium. Zur Durchführung eines alkalischen Phosphatasenachweises in Blutserum wird o-Kresolphthaleinmonophosphat-Dinatriumsalz zusammen mit Diethanolamin als Phosphorsäureakzeptor, welcher in sehr hoher Konzentration (ein molar) als Puffer mit Magnesiumchlorid vorliegt, eingesetzt. Dieses o-Kresolphthaleinmonophosphorsäure-SaIz ist jedoch nicht stabil gegenüber nicht-enzymatischer Hydrolyse. Dies bedeutet, daß bereits bei Abwesenheit des zu bestimmenden Enzyms o-Kresolphthaleinmonophosphat umgesetzt und Farbe gebildet wird. Die Empfindlichkeit des alkalischen Phosphatase-Nachweises sinkt dadurch, weil die Messung vor einem erhöhten Farbhintergrund durchgeführt werden muß oder es wird sogar die Anwesenheit tatsächlich nicht vorhandenen Enzyms vorgetäuscht.

In der Europäischen Patentanmeldung 0182179 wird dieses Problem aufgegriffen. Es wird dort empfohlen, zur Erhöhung der Stabilität von Nachweisreagenzien für alkalische Phosphatase Enzymsubstrat und Phosphorsäureakzeptor vor der Durchführung des Nachweises räumlich zu trennen und beide erst zusammen mit der zu untersuchenden Probenflüssigkeit in Kontakt zu bringen Hierfür wird ein mehrschichtiges Analysenelement beschrieben, bei dem sich das Substrat für alkalische Phosphatase und der Phosphorsäureakzeptor auf unterschiedlichen Schichten befinden Als mögliche alkalische Phosphatase-Substrate werden Arylphosphatammoniumsalze genannt, bei denen die Arylgruppe nitrosubstituiertes Phenyl, Phenolphthalein, Thymolphthalein, arylazosubstituiertes Aryl oder Fluorescein und die Ammoniumgruppe u. a. N-Methylglucamin sein kann. Als bevorzugte Phosphorsäureakzeptoren werden Aminoalkohole, darunter auch N-Methylglucamin genannt. o-Kresolphthaleinmonophosphate werden nicht als mögliche Phosphatase-Substrate aufgeführt. Ein wesentlicher Nachteil des Gegenstandes der europaischen Patentanmeldung 0182179 ist die Trennung von Substrat- und Phosphorsäureakzeptor Eine Aufbringung der jeweiligen Substanzen auf getrennte Schichten eines Analysenelementes ist für die Konstruktion des Teststreifens aufwendig. Außerdem ist auch zu berücksichtigen, daß jede Schicht Probenflüssigkeitsvolumen absorbiert. Gerade beim Einsatz von Analysenelementen ist die zur Verfügung stehende Probenflüssigkeitsmenge aber oft sehr klein und knapp. Außerdem können beim Aufgeben flüssiger Probe auf die getrennten Substrat- und Akzeptorschichten Probleme der homogenen Durchmischung von Substrat, Phosphorsäureakzeptor und Probe auftreten, die zu erheblichen Meßwertstreuungen führen können.

Die Aufgabe der vorliegenden Erfindung bestand deshalb darin, die Nachteile des Standes der Technik zu vermeiden.

Dies wird durch den Gegenstand der Erfindung, wie er in den Patentansprüchen charakterisiert ist, erreicht.

Gegenstand der Erfindung ist eine stabile Mischung zum alkalische Phosphatase-Nachweis, die N-Methylglucaminsalz der o-Kresolphthaleinmonophosphorsäure als Enzymsubstrat und N-Methylglucamin als Phosphorsäureakzeptor enthält. Beide liegen vorzugsweise homogen vermischt nebeneinander vor.

Ebenfalls Gegenstand der Erfindung ist ein Analysenelement zum Nachweis alkalischer Phosphatase, vorzugsweise in flüssiger Probe, enthaltend eine Matrix mit einem Substrat des Enzyms alkalische Phosphatase, das in Anwesenheit des Enzyms zu einer Farbänderung führt, wobei die Matrix die vorgenannte erfindungsgemäße Mischung trägt und somit sowohl das Enzymsubstrat als auch der Phosphorsäureakzeptor in oder auf einer Schicht vorliegen.

Weiter ist Gegenstand der Erfindung ein Verfahren zur Bestimmung von alkalischer Phosphatase mit einem Salz der o-Kresolphthaleinmonophosphorsäure als Enzymsubstrat und einem Phosphorsäureakzeptor, wobei die zu untersuchende Probe mit einer erfindungsgemäßen Mischung oder einem erfindungsgemäßen Analysenelement in Kontakt gebracht und bei Anwesenheit alkalischer Phosphatase eine Farbänderung beobachtet wird.

Salz der o-Kresolphthaleinmonophosphorsäure mit N-Methylglucamin ist neu. Ebenso das Verfahren zur Herstellung solchen Salzes aus o-Kresolphthaleinmonophosphorsäure und N-Methylglucamin.

Überraschenderweise hat es sich gezeigt, daß eine Mischung von N-Methylglucaminsalz der o-Kresolphthaleinmonophosphorsäure und zusätzlichem N-Methylglucamin als Phosphorsäureakzeptor die zur Bestimmung alkalischer Phosphatase verwendet werden kann, sehr stabil gegenüber nicht-enzymatischer Hydrolyse ist. Dies trifft auch zu, wenn die Mischung Puffersubstanz und alle übrigen für den alkalischen Phosphatasenachweis erforderlichen Substanzen enthält. Gerade die Anwesenheit von Puffersubstanz bzw. Phosphorsäureakzeptor ist in der europäischen Patentanmeldung 0182179 als eine wesentliche Ursache für die nicht-enzymatische Hydrolyse von Substraten des Enzyms alkalische Phosphatase angesehen worden. Als N-Methylglucaminsalz der o-Kresolphthaleinmonophosphorsäure werden vorteilhafterweise das D-N-Methylglucaminsalz und als Phosphorsäureakzeptor bevorzugt freies, d.h., zusätzlich zugegebenes D-N-Methylglucamin eingesetzt. Das Salz der o-Kresolphthaleinmonophosphorsäure kann 1 bis 3 N-Methylglucammoniumionen pro o-Kresolphthaleinmonophosphatmolekül enthalten. Vorzugsweise enthält das o-Kresolphthaleinmonophosphat der vorliegenden Erfindung 2 bis 3 N-Methylglucammoniumionen pro Molekül. In der besonders bevorzugten Ausführungsform enthält das o-Kresolphthaleinmonophosphorsäuresalz durchschnittlich 2,3 N-Methylglucammoniumionen pro o-Kresolphthaleinmonophosphatmolekül.

Die erfindungsgemäße Mischung ist auch in Anwesenheit von für den alkalischen Phosphatasenachweis notwendigem Natrium- oder/und Magnesiumsalz sowie in Anwesenheit von Puffersubstanz stabil gegen nicht-enzymatische Hydrolyse. Die Puffersubstanz liegt dabei in einer solchen Menge vor, daß für den Nachweis von alkalischer Phosphatase ein pH-Wert zwischen 10 und 12, vorzugsweise zwischen 10 und 11 sichergestellt ist. Mögliche Puffersubstanzen sind dem Fachmann bekannt. Lediglich als Beispiele, nicht als abschließende Aufzählung seien genannt: Carbonate, Borate, Phosphate, Good-Puffersubstanzen, Glutamat oder Aspartat. Da die Anwesenheit von Natrium- oder/und Magnesiumsalz bei der Bestimmung alkalischer Phosphatase vorteilhaft ist, kann beispielsweise die Puffersubstanz in Form solcher Salze vorliegen oder Natrium- oder/und Magnesiumsalz können der erfindungsgemäßen stabilen Mischung zusätzlich beigefügt sein.

Vorzugsweise soll zur Einstellung optimaler Bedingungen für die Enzymaktivität die erfindungsgemäße Mischung als Phosphorsäureakzeptor mindestens soviel N-Methylglucamin enthalten, daß Phosphorsäure, die bei Anwesenheit alkalischer Phosphatase aus dem Substrat abgespalten wird, durch den Phosphorsäureakzeptor abgefangen werden kann. Vorteilhafterweise wird die erfindungsgemäße stabile Mischung einen Überschuß an N-Methylglucamin enthalten. Das molare Verhältnis zwischen Enzymsubstrat und Phosphorsäureakzeptor wird mindestens 1 : 1 betragen, vorteilhafterweise aber bis zu 1 : 4 sein. Natürlich kann auch bei davon abweichenden Verhältnissen gearbeitet werden, wenn keine optimalen Enzymaktivitätsbedingungen erforderlich sind.

Obwohl Alkalisalz der o-Kresolphthaleinmonophosphorsäure sehr instabil gegenüber nicht-enzymatischer Hydrolyse ist, ist das N-Methylglucaminsalz auch in Gegenwart sehr hoher Konzentrationen an Alkaliionen stabil gegen nicht-enzymatische Hydrolyse.

Die erfindungsgemäße Mischung ist in Lösung sehr stabil. Ganz besonders stabil gegen nicht enzymatische Hydrolyse ist sie jedoch in trockener Form, d. h. als Mischung der trockenen Einzelsubstanzen, beispielsweise auch in lyophilisierter Form. Sie kann auch in oder auf einer Matrix vorliegen und dann Bestandteil eines Analysenelements zum Nachweis alkalischer Phosphatase sein. Als Matrix wird jedes schichtförmige Material bezeichnet, das in der Lage ist, die erfindungsgemäße Mischung zu tragen, ohne sie negativ zu beeinflussen. Als Matrixmaterialien kommen üblicherweise solche Materialien in Frage, die in der Lage sind, eine Lösung der erfindungsgemäßen stabilen Mischung durch Quellung oder Absorption aufzunehmen, beispielsweise durch Imprägnierung, oder solche die mit der Mischung beschichtet werden können oder die in Gegenwart der erfindungsgemäßen Mischung hergestellt werden können und deshalb die Mischung in das Matrixmaterial selbst einlagern. Letzteres ist beispielsweise möglich bei der Herstellung von Filmschichten aus filmbildenden Materialien, die in Gegenwart der Mischungsbestandteile vorliegen. Im Rahmen der vorliegenden Erfindung sind Analysenelemente besonders bevorzugt, die eine saugfähige, poröse Matrix enthalten, die mit einer Lösung der erfindungsgemäßen Mischung imprägniert und anschließend getrocknet worden ist. Als besonders bevorzugtes Matrixmaterial wird Papier verwendet.

Die die erfindungsgemäße Mischung tragende Matrix kann bereits selbst als Analysenelement verwendet werden. Es kann sich aber auch als vorteilhaft erweisen, wenn die Matrix beispielsweise auf einem inerten Trägermaterial befestigt ist, die die Handhabung der Matrix erleichtert. Ähnliche Ausführungen sind beispielsweise für Urinteststreifen bekannt. Die Matrix mit der erfindungsgemäßen stabilen Mischung kann auch in mehrschichtigen Analysenelementen enthalten sein, wie sie aus dem Stand der Technik bekannt sind. Beispielsweise kann sie in einem mehrschichtigen Analysenelement enthalten sein, wie sie in der europäischen Patentanmeldung 0182179 beschrieben ist, wobei die Matrix die Spreit- und Pufferschicht dieses Analysenelements ersetzt.

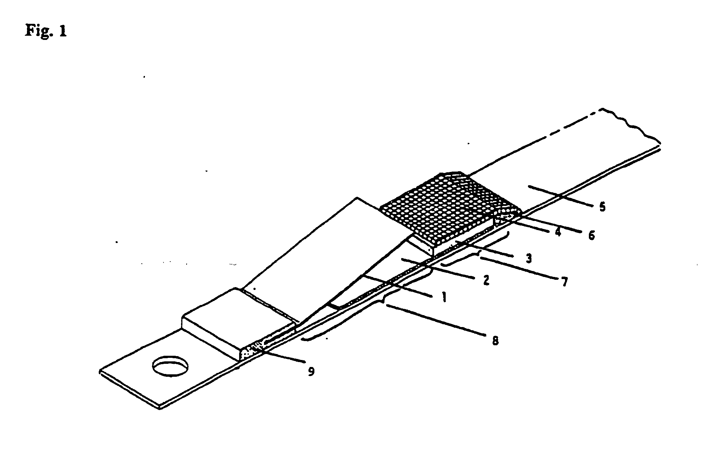

Für die Untersuchung von Vollblut hat es sich als besonders vorteilhaft erwiesen, ein Analysenelement zu verwenden, wie es beispielsweise in der europäischen Patentanmeldung 0461392 am Beispiel der Figur 1 beschrieben ist. Auf einer innerten Trägerfolie (5), beispielsweise einer Kunststoff-Folie, ist eine Transportschicht (2) befestigt, die zum Transport von Probeflüssigkeit aus der Probenaufgabezone (7) in den Nachweisbereich (8) dient. Als Transportschicht (2) ist prinzipiell jedes Material geeignet, das die zu untersuchende Flüssigkeit aus der Probenaufgabezone (7) in den Nachweisbereich (8) zu transportieren in der Lage ist und sie dabei nicht so verändert, daß die Analyse beeinträchtigt wird. Besonders vorteilhaft ist es, als Transportschicht (2) ein Glasfaserflies einzusetzen. Die Transportschicht (2) teilweise überdeckend ist eine Schicht (3) zur Abtrennung von korpuskulären Bestandteilen aus der Probenflüssigkeit befestigt. Grundsätzlich kann hierfür jedes Material verwendet werden, das erlaubt, korpuskuläre Bestandteile aus der Probenflüssigkeit, insbesondere Blutzellen, vor allem Erythrozyten aus Blut, abzutrennen und diese nicht in wesentlichen Mengen in den Nachweisbereich (8) gelangen zu lassen, so daß sie dort keine Störung der Nachweisreaktion verursachen. Im übrigen darf die Abtrennschicht (3) nicht zu einer Veränderung der Probenflüssigkeit dahingehend führen, daß darin die Aktivität der zu bestimmenden alkalischen Phosphatase verändert und so das Ergebnis verfälscht wird. Als besonders geeignet für die Abtrennschicht (3) haben sich Glasfaserfliese erwiesen, wie sie z. B. in der europäischen Patentschrift 0 045 476 beschrieben sind. Über der Abtrennschicht (3) ist vorteilhafterweise eine Schutzschicht (4) angebracht, die verhindern soll, daß bei der Probenaufgabe, beispielsweise mittels einer Pipette, die Abtrennschicht (3) beschädigt wird. Bewährt hat sich hierfür ein Netz aus innertem Material, beispielsweise aus Kunststoff. Schutzschicht (4) und Abtrennschicht (3) sind auf der inerten Trägerfolie (5) befestigt. Dies kann beispielsweise mittels eines Schmelzklebestreifens (6) erfolgen.

Seitlich von der Transportschicht (2) ist eine aus durchsichtigem Kunststoff bestehende Trägerfolie mit der Matrix (1), die die erfindungsgemäße Mischung enthält, befestigt. Vorteilhafterweise geschieht dies über eine Klebestelle (9), beispielsweise einen Schmelzkleberstreifen. Die Matrix (1) ist so angeordnet, daß sie beim Niederdrücken der durchsichtigen Trägerfolie in Richtung der innerten Trägerfolie (5) mit der Transportschicht (2) in einen einen Flüssigkeitsübergang-ermöglichenden Kontakt gebracht werden kann.

Zur Durchführung des erfindungsgemäßen Verfahrens zur Bestimmung von alkalischer Phosphatase in flüssiger Probe, wird die Probe mit einer erfindungsgemäßen Mischung in Kontakt gebracht, woraufhin bei Anwesenheit alkalischer Phosphatase eine Farbänderung beobachtet werden kann. In der Regel wird alkalische Phosphatase in Körperflüssigkeiten, wie Blut, oder von Blut abgeleiteten Proben, wie Plasma oder Serum, nachgewiesen. Natürlich ist es aber auch möglich, andere Flüssigkeiten auf die Anwesenheit alkalischer Phosphatase zu untersuchen. Grundsätzlich ist es möglich, die erfindungsgemäße Mischung zu der zu untersuchenden Probe zu geben oder umgekehrt die Probe zu der erfindungsgemäßen Mischung hinzuzufügen. Für den Fall, daß die erfindungsgemäße Mischung trägerfrei vorliegt, d. h. als Pulver, Kristallmischung oder in lyophilisierter Form, wird es wohl in den meisten Fällen vorteilhaft sein, die Mischung zu der zu untersuchenden Probe zu geben.

Für den Fall, daß ein erfindungsgemäßes Analysenelement mit einer die erfindungsgemäße Mischung tragenden Matrix zum Einsatz gelangt, ist es vorteilhaft, daß die Matrix mit der zu untersuchenden Flüssigkeit in Kontakt gebracht wird. Beispielsweise wird die Matrix in die zu untersuchende Flüssigkeit eingetaucht. Für eine besonders bevorzugte Durchführung der Bestimmung alkalischer Phosphatase mit einem erfindungsgemäßen Analysenelement wird Vollblut auf die Schutzschicht (4) des in Figur 1 dargestellten besonders bevorzugten Analysenelementes gegeben. Das Blut dringt in die Abtrennschicht (3) ein und Erythrozyten werden von Plasma bzw. Serum getrennt. Durch Kapillarkräfte wird die so gewonnene Flüssigkeit in die Nachweiszone (8) gesaugt. Durch Druck auf die Trägerfolie mit der erfindungsgemäßen Matrix (1) wird die wässrige Phase in der Transportschicht (2) mit der Matrix (1) in Kontakt gebracht, Flüssigkeit dringt in die Matrix ein und die Bestimmungsreaktion wird ausgelöst. Die Reaktion wird anhand der Verfärbung in der Matrix (1) durch die Trägerfolie der Matrix (1) hindurch visuell beobachtet oder reflexionsphotometrisch gemessen.

Salz der o-Kresolphthaleinmonophosphorsäure mit N-Methylglucamin, wie es als Bestandteil der erfindungsgemäßen stabilen Mischung beschrieben ist, ist neu. Zu seiner Herstellung wird o-Kresolphthaleinmonophosphorsäure mit N-Methylglucamin umgesetzt. Die Herstellung von o-Kresolphthaleinmonophosphorsäure ist dem Fachmann aus dem US-Patent 3,975,405 bekannt. N-Methylglucamin ist käuflich erhältlich. Vorteilhafterweise wird D-N-Methylglucamin eingesetzt.

Zur Umsetzung der o-Kresolphthaleinmonophosphorsäure mit N-Methylglucamin erfolgt vorteilhafterweise so, daß die o-Kresolphthaleinmonophosphorsäure in organischem Lösungsmittel gelöst und mit zwei bis vier Äquivalenten N-Methylglucamin versetzt wird. Das ausgefallene Salz wird abfiltriert und durch Umkristallisation aus einem geeigneten organischen Lösungsmittel gereinigt. Für das organische Lösungsmittel als Reaktionsmedium haben sich Alkohole, wie Ethanol und bevorzugt Methanol, erwiesen. Zur Umkristallisation kommen unpolare Lösungsmittel in Frage, wie beispielsweise Ether. Das auf die vorstehende Art und Weise gewonnene Salz der o-Kresolphthaleinmonophosphorsäure enthält zwischen zwei und drei N-Methylglucammoniumionen pro o-Kresolphthaleinmonophosphorsäure-Molekül.

Neues o-Kresolphthaleinmonophosphorsäuresalz hat sich für den Nachweis alkalischer Phosphatase als sehr vorteilhaft erwiesen, da das enzymatische Spaltprodukt o-Kresolphthalein einen hohen molaren Extinktionskoeffizienten aufweist und so sehr empfindliche Bestimmungen möglich sind.

Dadurch, daß das gleiche organische Amin als Kation des Enzymsubstrats und als Phosphorsäureakzeptor eingesetzt wird, gestaltet sich die Herstellung der erfindungsgemäßen Mischung zum alkalischen Phosphatasenachweis sehr einfach. Darüber hinaus ist diese Mischung sehr stabil gegen nicht-enzymatische Hydrolyse. Dies führt dazu, daß solche Mischungen ohne nennenswerte Mengen von Hydrolyseprodukten hergestellt werden können, was zu niedrigen Ausgangssignalen bei Meßungen führt und außerdem sind solche Mischungen sehr lagerstabil. Die Anwesenheit von Alkaliionen, wie beispielsweise Natriumionen sogar in großem Überschuß beeinflußt die Stabilität der erfindungsgemäßen Mischung nicht. Da ein erfindungsgemäßes Analysenelement eine gemeinsame Schicht für Enzymsubstrat und Phosphorsäureakzeptor besitzt, ist die Herstellung einfach. Darüber hinaus kommt das erfindungsgemäße Analysenelement mit wenig Probenflüssigkeit aus, weil statt der empfohlenen Zweischichtigkeit des Standes der Technik nur eine Schicht mit erfindungsgemäßer Mischung Flüssigkeit aufnehmen muß. Darüber hinaus führt die Verwendung nur einer Schicht, die Substrat und Akzeptor bereits vermischt enthält, bei Probenaufgabe zu einer homogenen Mischung, die zu reproduzierbaren Meßergebnissen führt.

Die Erfindung wird durch die nachfolgenden Beispiele näher erläutert.

### Beispiel 1

### Herstellung von o-Kresolphthaleinphosphat-N-Methylglucaminsalz

o-Kresolphthaleinmonophosphorsäure wird gemäß US-Patent 3,975,405 hergestellt. 24 g o-Kresolphthaleinphosphorsäure werden in 94 ml Methanol gelöst und hierzu 23 g D-N-Methylglucamin zugefügt. Das N-Methylglucammoniumsalz wird durch Fällung aus dem 10fachen Volumen Diäthyläther isoliert. Der Niederschlag wird im Vakuum über Schwefelsäure getrocknet. Ausbeute: 44 g o-Kresolphthaleinphosphat-D-N-Methylglucammoniumsalz.

### Beispiel 2

### Herstellung von o-Kresolphthaleinphosphat-Natriumsalz

24 g o-Kresolphthaleinphosphorsäure werden in 94 ml gelöst, der pH-Wert der Lösung mit NaOH auf 10,5 gestellt und im 10fachen Volumen Diäthyläther ausgefällt. Der Niederschlag wird abgetrennt und im Vakuum über Schwefelsäure getrocknet. Ausbeute: 22 g o-Kresolphthaleinphosphat-Natriumsalz.

### Beispiel 3

### Nachweis von alkalischer Phosphatase

Papier (Langfaserpapier, Hersteller Schoeller, Gernsbach, Deutschland, Flächengewicht 12 g / m²) wird mit einer erfindungsgemäßen Mischung imprägniert, die folgendermaßen hergestellt wird:
- Wasser, gereinigt 500,0 ml
- Natronlauge (5N) 91,0 ml
- D-N-Methylglucamin 187,4 g
- L-Asparaginsäure-Magnesiumsalz 22,0 mg
- o-Kresolphthaleinphosphorsäure-D-N-Methylglucaminsalz 218,1 g
werden gemischt und anschließend mit Wasser auf 1 l aufgefüllt.

Der pH-Wert der Lösung wird gegebenenfalls mit Natronlauge (5N) auf pH 11.0 korrigiert.

Nach Trocknung des imprägnierten Papiers wird Blutserum, das alkalische Phosphatase enthält, auf das Papier aufgegeben. Es findet in Abhängigkeit von der Enzymaktivität eine Farbänderung von gelb nach rot-violett statt.

### Beispiel 4

### Stabilitätsvergleich

Ein wie in Beispiel 3 beschrieben hergestelltes Papier mit der erfindungsgemäßen Mischung zum alkalischen Phosphatasenachweis wird über mehrere Wochen bei 50 °C gelagert. Nach bestimmten Zeitabständen wird die Extinktion bei 567 nm gegen Wasser gemessen. Mit dem in Beispiel 2 hergestellten o-Kresolphthaleinmonophosphat-Natriumsalz wird gemäß Beispiel 3 anstelle des erfindungsgemäßen
o-Kresolphthaleinmonophosphorsäure-N-Methylglucammoniumsalzes eine Mischung zum alkalischen Phosphatase-Nachweis hergestellt und auf das gleiche Langfaserpapier imprägniert und getrocknet, so daß sich ein dem in Beispiel 3 beschriebenen analoges Analyseelement ergibt. Dieses Papier wird den gleichen Lagerbedingungen unterworfen wie das Methylglucaminsalz-haltige.

Die Ergebnisse nach eineinhalb und drei Wochen sind Tab. 1 zu entnehmen.

**Tabelle 1**

| Belastungsdauer | Extinktion des Papiers mit Natriumsalz | Extinktion des Papiers mit N-Methylglucammoniumsalz |
|---|---|---|
| 1,5 Wochen | 2,664 | 1,022 |
| 3 Wochen | 3,735 | 1,255 |

Hieraus ergibt sich, daß das Natriumsalz wesentlich instabiler als das N-Methylglucammoniumsalz gegenüber nicht enzymatischer Hydrolyse ist.

## Patentansprüche

1. Gegen nicht-enzymatische Hydrolyse stabile Mischung zum alkalischen Phosphatasenachweis **dadurch gekennzeichnet, daß** sie N-Methylglucaminsalz der o-Kresolphthaleinmonophosphorsäure als Enzymsubstrat und N-Methylglucamin als Phosphorsäureakzeptor enthält.

2. Stabile Mischung gemäß Patentanspruch 1, **dadurch gekennzeichnet, daß** das Salz des D-N-Methylglucamins und freies D-N-Methylglucamin vorliegen.

3. Stabile Mischung gemäß Patentansprüchen 1 oder 2, **dadurch gekennzeichnet, daß** es sich bei dem Salz um ein solches mit 2 - 3 N-Methylglucammoniumionen pro o-Kresolphthaleinmonophosphat-molekül handelt.

4. Stabile Mischung gemäß einem der Patentansprüche 1 bis 3, **dadurch gekennzeichnet, daß** sie in trockener Form vorliegt.

5. Analysenelement zum Nachweis alkalischer Phosphatase, enthaltend eine Matrix mit einem Substrat des Enzyms alkalische Phosphatase, das in Anwesenheit des Enzyms zu einer Farbänderung führt, **dadurch gekennzeichnet, daß** die Matrix eine Mischung gemäß einem der Patentansprüche 1-4 trägt, und somit sowohl das Enzymsubstrat als auch der Phosphorsäureakzeptor in oder auf einer Schicht vorliegen.

6. Verfahren zur Bestimmung von alkalischer Phosphatase mit einem Salz der o-Kresolphthaleinmonophosphorsäure als Enzymsubstrat und einem Phosphorsäureakzeptor, **dadurch gekennzeichnet, daß** die zu untersuchende Probe mit einer Mischung gemäß einem der Patentansprüche 1-4 oder einem Analysenelement gemäß Patentanspruch 5 in Kontakt gebracht und bei Anwesenheit alkalischer Phosphatase eine Farbänderung beobachtet wird.

## Claims

1. Mixture for the detection of alkaline phosphatase that is stable towards non-enzymatic hydrolysis, wherein it contains the N-methyl-glucamine salt of o-cresolphthalein monophosphoric acid as the enzyme substrate and N-methylglucamine as the phosphoric acid acceptor.

2. Stable mixture as claimed in claim 1, wherein the D-N-methylglucamine salt and free D-N-methylglucamine are present.

3. Stable mixture as claimed in claims 1 or 2, wherein the salt is one containing 2-3 N-methyl-glucammonium ions per o-cresolphthalein monophosphate molecule.

4. Stable mixture as claimed in one of the claims 1 to 3, wherein it is present in a dry form.

5. Analytical element for the detection of alkaline phosphatase containing a matrix with a substrate of the enzyme alkaline phosphatase which leads to a change in colour when the enzyme is present, wherein the matrix carries a mixture as claimed in one of the claims 1 to 4 and thus the enzyme substrate as well as the phosphoric acid acceptor are present in or on one layer.

6. Method for the determination of alkaline phosphatase using a salt of o-cresolphthalein monophosphoric acid as the enzyme substrate and a phosphoric acid acceptor, wherein the sample to be examined is brought into contact with a mixture as claimed in one of the claims 1 - 4 or an analytical element as claimed in claim 5 and a change in colour is observed when alkaline phosphatase is present.

## Revendications

1. Mélange stable à l'hydrolyse non enzymatique pour la mise en évidence de la phosphatase alcaline, **caractérisé en ce qu'**il contient le sel de N-méthylglucamine de l'acide o-crésolphtaléinemonophosphorique comme substrat enzymatique et de la N-méthylglucamine comme accepteur d'acide phosphorique.

2. Mélange stable selon la revendication 1, **caractérisé en ce que** le sel de la D-N-méthylglucamine et la D-N-méthylglucamine libre sont présents.

3. Mélange stable selon les revendications 1 ou 2, **caractérisé en ce qu'**il s'agit, pour le sel, d'un sel avec 2 à 3 ions N-méthylglucammonium par molécule d'o-crésolphtaléinemonophosphate.

4. Mélange stable selon l'une quelconque des revendications 1 à 3, **caractérisé en ce qu'**il se trouve sous forme sèche.

5. Elément d'analyse pour la mise en évidence de la phosphatase alcaline, contenant une matrice avec un substrat de l'enzyme phosphatase alcaline, qui conduit, en présence de l'enzyme, à une modification de couleur, **caractérisé en ce que** la matrice porte un mélange selon l'une quelconque des revendications 1 à 4 et que tant le substrat enzymatique que l'accepteur d'acide phosphorique se trouvent dans ou sur une couche.

6. Procédé pour déterminer la phosphatase alcaline avec un sel de l'acide o-crésolphtaléinemonophosphorique comme substrat enzymatique et un accepteur d'acide phosphorique, **caractérisé en ce que** l'échantillon à analyser est mis en contact avec un mélange selon l'une quelconque des revendications 1 à 4 ou un élément d'analyse selon la revendication 5 et on observe une modification de couleur en présence de la phosphatase alcaline.
